## Europäisches Patentamt
## European Patent Office
## Office européen des brevets

(11) Veröffentlichungsnummer: **0 046 894**
**B1**

(12) **EUROPÄISCHE PATENTSCHRIFT**

(45) Veröffentlichungstag der Patentschrift:
18.07.84

(21) Anmeldenummer: 81106188.6

(22) Anmeldetag: 07.08.81

(51) Int. Cl.³: **A 61 F 7/10**, A 61 F 7/00

(54) Packungskissen für die Thermotherapie, insbesondere Kältetherapie.

(30) Priorität: 01.09.80 DE 3032884

(43) Veröffentlichungstag der Anmeldung:
10.03.82 Patentblatt 82/10

(45) Bekanntmachung des Hinweises auf die Patenterteilung:
18.07.84 Patentblatt 84/29

(84) Benannte Vertragsstaaten:
AT CH FR GB LI NL SE

(56) Entgegenhaltungen:
DE - A - 1 205 566
GB - A - 1 425 591
US - A - 3 301 250
US - A - 3 865 117

**Die Akte enthält technische Angaben, die nach dem Eingang der Anmeldung eingereicht wurden und die nicht in dieser Patentschrift enthalten sind.**

(73) Patentinhaber: **Rödler GmbH, Rödlerstrasse, D-6521 Flörsheim-Dalsheim (DE)**

(72) Erfinder: **Francas, Gernot, Dr., Weinsheimer Strasse 75, D-6520 Worms (DE)**

(74) Vertreter: **Katscher, Helmut, Dipl.-Ing., Bismarckstrasse 29, D-6100 Darmstadt (DE)**

BUNDESDRUCKEREI BERLIN

**Beschreibung**

Die Erfindung betrifft ein Packungskissen für die Thermotherapie, insbesondere Kältetherapie, mit einer aus flexibler Folie bestehenden, dicht verschweißten Packungshülle und einer Packungsfüllung mit einer wärme- bzw. kältespeichernden Flüssigkeit und einem Einlagematerial.

Derartige Packungskissen werden in der Thermotherapie verwendet, um auf die zu behandelnden Körperteile des Patienten Kälte oder Wärme zu übertragen. Hierzu werden die Packungskissen erwärmt, beispielsweise im Wasserbad, oder gekühlt, beispielsweise in einer Kühltruhe, und dann auf die zu behandelnden Körperteile aufgelegt, beispielsweise auf ein Gelenk. Die Packungskissen sollen einerseits ein gutes Wärmespeichervermögen haben; andererseits sollen sie sich der Körperoberfläche gut anpassen. Deshalb ist es erforderlich, daß die Packungskissen auch bei starker Unterkühlung flexibel bleiben. Deshalb wird als Füllung nicht nur Wasser, sondern eine Glykol-Wasser-Mischung verwendet.

Ein nur mit einer Flüssigkeit gefülltes Packungskissen führt bei der Auflage auf die unebene Oberfläche des zu behandelnden Körperteils zu einer ungleichmäßigen Wärme- bzw. Kälteübertragung, weil sich die Flüssigkeit an den tieferliegenden Stellen des Packungskissens konzentriert. Deshalb ist schon vorgeschlagen worden, als Einlagematerial eine Schaumstoffeinlage zu verwenden, die mit der Flüssigkeit getränkt ist. Da die Wärmeübertragung sowohl bei der Vorbereitung des Packungskissens als auch bei der Anwendung am Körper des Patienten nur an der Oberfläche erfolgen kann, wird die Wärmeübertragung wesentlich verbessert, wenn hierfür nicht nur die Wärmeleitung innerhalb der Flüssigkeit, sondern auch eine durch den Wärmeübertragungsvorgang unterstützte Konvektion erfolgt. Diese Konvektion ist aber nahezu vollständig unterbunden, wenn die Flüssigkeit in einem Schaumstoffkörper aufgesaugt ist.

Durch die vorgegebene Form des Schaumstoffkörpers ist die Modulierbarkeit des Packungskissens, d. h. die Anpassung an die Körperoberfläche, eingeschränkt. Da die Temperaturabgabe ungehindert erfolgt, tritt häufig eine zu starke und zu rasche Wärmeübertragung auf, d. h. der Körperteil wird zu stark gekühlt oder erwärmt, wobei diese Wirkungen einerseits zu rasch abklingen und andererseits zu unliebsamen Erscheinungen führen können (Verbrennungen, Erfrierungen).

Aufgabe der Erfindung ist es daher, ein Packungskissen der eingangs genannten Art so zu gestalten, daß bei guter mechanischer Modulierbarkeit zur Anpassung an die Körperoberfläche eine gleichmäßige, dosierte Wärme- bzw. Kälteübertragung sichergestellt ist.

Diese Aufgabe wird erfindungsgemäß dadurch gelöst, daß das Einlagematerial aus einer losen Schüttung von geschlossenporigen Schaumstoffkörpern besteht. Diese Schaumstoffkörper, die sich nicht mit der Flüssigkeit vollsaugen, sondern ihre isolierende Eigenschaft beibehalten, verbessern die Modulierbarkeit des Packungskissens auch gegenüber einem nur mit Flüssigkeit gefüllten Kissen wesentlich, weil sie eine gleichmäßige Verteilung der Flüssigkeit im Packungskissen über die gesamte zu behandelnde Oberfläche sicherstellen, und zwar auch, wenn die Oberfläche stark gekrümmt ist, beispielsweise bei der Anwendung am Knie. Die Wärmeübertragung, durch die die Haut- bzw. Gewebetemperatur angehoben oder gesenkt wird, erfolgt dosiert, weil die Länge des für die Wärmeübertragung bedeutsamen Weges, der durch Wärmeleitung zurückgelegt werden muß, durch die in der Flüssigkeit verteilten, isolierenden Schaumstoffkörper vergrößert ist. Die Konvektion in der Flüssigkeit wird nicht unterbunden, jedoch gebremst, so daß über einen längeren Zeitraum eine gleichmäßige Wärmeabgabe (Hebung bzw. Senkung der Hauttemperatur) erreicht wird. Die Oberflächentemperatur des Packungskissens auf der Haut bleibt über eine verhältnismäßig lange Zeitspanne im wesentlichen konstant.

Da die Schaumstoffkörper leichter sind als die sie umgebende Flüssigkeit, konzentrieren sich die Schaumstoffkörper im Ruhezustand etwas stärker an der Oberseite des Packungskissens. Dadurch tritt an der Oberseite, die normalerweise nicht mit dem zu behandelnden Körperteil in Berührung steht, eine gewisse Isolierwirkung auf, die einen Wärme- bzw. Kälteverlust an der Oberseite des Packungskissens verringert.

In besonders vorteilhafter Ausgestaltung des Erfindungsgedankens ist vorgesehen, daß die Schaumstoffkörper aus Polystyrol bestehen. Dieser in einfacher Weise zu Schaumstoffkörpern verarbeitbare Kunststoff hat sich durch seine Temperaturunempfindlichkeit und sein neutrales Verhalten gegenüber allen infrage kommenden Flüssigkeiten als besonders geeignet erwiesen. Bei ausreichender Formbeständigkeit sind Polystyrol-Schaumstoffkörper dabei aber weich genug, um Beschädigungen der Packungshülle auch dann auszuschließen, wenn das Packungskissen mechanisch beansprucht, beispielsweise zwischen harten Oberflächen eingeklemmt wird.

Vorzugsweise sind die Schaumstoffkörper Schaumstoffkügelchen, weil sie in dieser Form gegeneinander besonders leicht bewegbar sind.

Vorzugsweise, jedoch nicht notwendig, liegen die Schaumstoffkügelchen in einem Durchmesserbereich von etwa 1—5 mm.

In weiterer Ausgestaltung des Erfindungsgedankens ist vorgesehen, daß die Packungshülle auf einer Seite metallisiert ist. Dadurch wird die Wärmeabgabe an dieser Seite, die nicht zur Anlage am zu behandelnden Körperteil kommt, wesentlich herabgesetzt.

Die Erfindung wird nachfolgend an einem Ausführungsbeispiel näher erläutert, das in der Zeichnung dargestellt ist. Es zeigt

Fig. 1 ein Packungskissen im Schnitt, das auf einen zu behandelnden Körperteil aufgelegt ist, und

Fig. 2 eine abgewandelte Ausführungsform des Packungskissens in einem vergrößerten Teilschnitt.

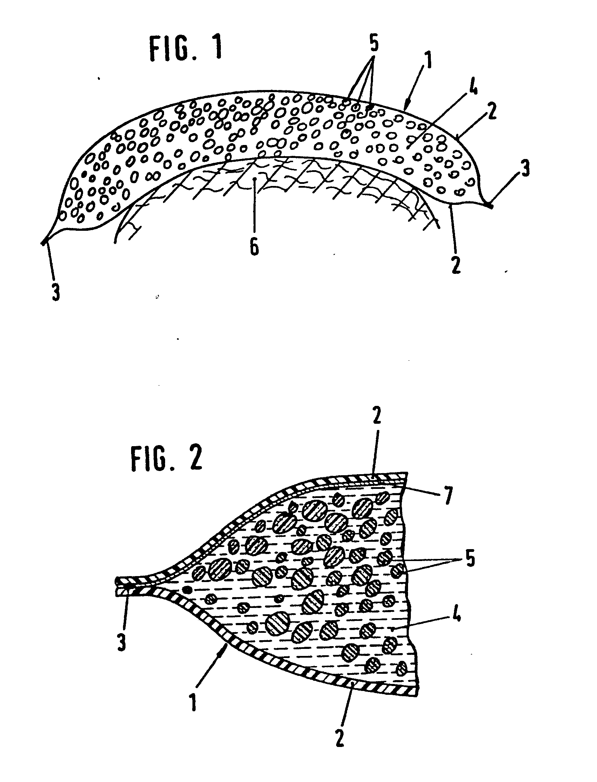

Das in Fig. 1 gezeigte Packungskissen 1 weist eine Packungshülle 2 auf, die aus einer flexiblen Verbundfolie aus Polyamid und Polyäthylen besteht. Die aus zwei Flächenstücken bestehende Packungshülle 2 ist an einer Randschweißung 3 dicht verschlossen. Die für die Packungshülle 2 verwendete Verbundfolie ist reißfest, hat eine hohe Widerstandsfähigkeit gegen Knickbeanspruchung und eine hohe Thermostabilität und ist gegen kurzwellige UV-Strahlung beständig, wie sie oft zur Desinfektion derartiger Packungskissen verwendet wird.

Die in der Packungshülle 2 enthaltene Packungsfüllung besteht aus einer wärmespeichernden Flüssigkeit 4, die gekühlt oder erwärmt werden kann, in der sich eine lose Schüttung von Polystyrol-Schaumstoffkügelchen 5 befindet. Die Flüssigkeit 5 besteht beispielsweise aus einer Glykol-Wasser-Mischung, die mit einem Thixotropierungsmittel auf Zellulosebasis angedickt ist. Außerdem kann ein Farbstoff zugegeben sein.

Die Schaumstoffkügelchen 5 haben im Durchschnitt einen Durchmesser von 2—4 mm und sind in solcher Menge zugegeben, daß sie das Packungskissen 1 weitgehend füllen, wobei jedoch noch eine ausreichende Bewegungsfreiheit für die Verformung des Packungskissens 1 zur Anpassung an den zu behandelnden Körperteil 6 gegeben ist.

Wie in Fig. 1 angedeutet, sammeln sich die Schaumstoffkügelchen 5, die spezifisch leichter sind als die sie umgebende Flüssigkeit 4, etwas stärker an der Oberseite des Packungskissens 1, ohne daß jedoch eine so weitgehende Entmischung eintreten würde, daß an der am Körperteil 6 anliegenden Unterseite des Packungskissens keine Schaumstoffkügelchen 5 mehr vorhanden wären.

Anstelle der beim gezeigten Ausführungsbeispiel verwendeten Polystyrol-Schaumstoffkügelchen können auch andere geschlossenporige Schaumstoffkörper verwendet werden.

Bei der in Fig. 2 gezeigten abgewandelten Ausführungsform des Packungskissens 1 ist an der oberen Wand der Packungshülle 2 eine metallisierte Schicht 7 aufgebracht, beispielsweise eine Metallfolie oder eine aufgespritzte oder aufgedampfte Schicht, die durch ihre Reflexionswirkung die Wärmeaufnahme von oben wesentlich herabsetzt. Die metallisierte Schicht 7 kann an der Außenseite oder Innenseite der Packungshüllenwand vorgesehen sein.

Bei der Anwendung in der Kältetherapie wird das Packungskissen 1 beispielsweise in einer Kühltruhe auf die gewünschte Temperatur unterkühlt und dann auf den zu behandelnden Körperteil 6 aufgelegt. Die Kälteübertragung erfolgt durch die Wirkung der Schaumstoffkügelchen 5 dosiert und über einen längeren Zeitraum gleichmäßig. Entsprechend ist die Wirkung bei der Anwendung in der Wärmetherapie, wobei das Packungskissen 1 beispielsweise im Wasserbad auf die gewünschte Temperatur gebracht wird.

**Patentansprüche**

1. Packungskissen (1) für die Thermotherapie, insbesondere Kältetherapie, mit einer aus flexibler Folie bestehenden, dicht verschweißten Packungshülle (2) und einer Packungsfüllung mit einer wärme- bzw. kältespeichernden Flüssigkeit (4) und einem Einlagematerial, dadurch gekennzeichnet, daß das Einlagematerial aus einer losen Schüttung von geschlossenporigen Schaumstoffkörpern (5) besteht.

2. Packungskissen nach Anspruch 1, dadurch gekennzeichnet, daß die Schaumstoffkörper (5) aus Polystyrol bestehen.

3. Packungskissen nach Anspruch 1, dadurch gekennzeichnet, daß die Schaumstoffkörper Schaumstoffkügelchen (5) sind.

4. Packungskissen nach Anspruch 2, dadurch gekennzeichnet, daß die Schaumstoffkügelchen (5) in einem Durchmesserbereich von etwa 1— 5 mm liegen.

5. Packungskissen nach Anspruch 1, dadurch gekennzeichnet, daß die Packungshülle (2) auf einer Seite eine metallisierte Schicht (7) aufweist.

**Claims**

1. Compress pack (1) for thermotherapy, in particular cryotherapy, comprising a fluidtightly welded compress cover (2) consisting of a flexible sheet, and a compress filling with a heat or cold-storing fluid (4), and an insert material, characterised in that the insert material consists of a loose fill of closed-pore foam-material bodies (5).

2. Compress pack according to claim 1, characterised in that the foam-material bodies (5) consist of polystyrene.

3. Compress pack according to claim 1, characterised in that the foam-material bodies are foam-material spherules (5).

4. Compress pack according to claim 2, characterised in that the foam-material spherules (5) have diameters in a range of from about 1—5 mm.

5. Compress pack according to claim 1, characterised in that, on one side, the compress cover (2) has a metallised layer (7).

**Revendications**

1. Coussinet (1) pour la thermothérapie, notamment la cryothérapie, comportant une enveloppe (2) consistant en une feuille flexible soudée de manière étanche et un remplissage constitué par un liquide (4) de stockage de la chaleur ou du froid et par une matière de chargement, caractérisé en ce que la matière de chargement consiste en des corps de matière alvéolaire (5) à pores fermés déversés en vrac.

2. Coussinet suivant la revendication 1, caractérisé en ce que les corps de matière alvéolaire (5) sont en polystyrène.

3. Coussinet suivant la revendication 1, caractérisé en ce que les corps de matière alvéolaire (5) sont des billes en matière alvéolaire.

4. Coussinet suivant la revendication 2, caractérisé en ce que les billes en matière alvéolaire (5) présentent un diamètre de l'ordre d'environ 1 à 5 mm.

5. Coussinet suivant la revendication 1, caractérisé en ce que l'enveloppe (2) présente, sur une face, une couche métallisée (7).

FIG. 1

FIG. 2